# EUROPEAN PATENT APPLICATION

(11) **EP 3 011 976 A1**
(43) Date of publication of application: **27.04.2016**
(21) Application number: 14003570.0
(22) Date of filing: 20.10.2014
(51) Int. Cl.: A61K 51/06

(54) **18F-tagged inhibitors of prostate specific membrane antigen (PSMA), their use as imaging agents**

(71) Applicant: Deutsches Krebsforschungszentrum, 69120 Heidelberg (DE); Ruprecht-Karls-Universität Heidelberg, 69117 Heidelberg (DE)
(72) Inventor: Kopka, Klaus, 69221 Dossenheim (DE); Benesova Martina, 69121 Heidelberg (DE); Cardinale Jens, 68259 Mannheim (DE); Eder, Matthias, 68239 Mannheim (DE); Schäfer, Martin, 69239 Neckarsteinach (DE); Bauder-Wüst, Ulrike, 69198 Schriesheim (DE); Haberkorn, Uwe, 68723 Schwetzingen (DE); Eisenhut, Michael, 69118Heidelberg (DE)
(74) Representative: Schüssler, Andrea

(57) **Abstract**

The present invention generally relates to the field of radiopharmaceuticals and their use in nuclear medicine as tracers, imaging agents and for the treatment of various disease states of prostate cancer.

## Description

The present invention generally relates to the field of radiopharmaceuticals and their use in nuclear medicine as tracers, imaging agents and for the treatment of various disease states of prostate cancer.

### BACKGROUND OF THE INVENTION

Prostate cancer (PCa) is the leading cancer in the US and European population. At least 1-2 million men in the western hemisphere suffer from prostate cancer and it is estimated that the disease will strike one in six men between the ages of 55 and 85. There are more than 300.000 new cases of prostate cancer diagnosed each year in USA. The mortality from the disease is second only to lung cancer. Currently anatomic methods, such as computed tomography (CT), magnetic resonance (MR) imaging and ultrasound, predominate for clinical imaging of prostate cancer. An estimated $ 2 billion is currently spent worldwide on surgical, radiation, drug therapy and minimally invasive treatments. However, there is presently no effective therapy for relapsing, metastatic, androgen-independent prostate cancer.

A variety of experimental low molecular weight PCa imaging agents are currently being pursued clinically, including radiolabeled choline analogs [¹⁸F]fluorodihydrotestosterone ([¹⁸F]FDHT), anti-1-amino-3-[¹⁸F]fluorocyclobutyl-1-carboxylic acid (anti[18F]F-FACBC, [¹¹C]acetate and1-(2-deoxy-2-[¹⁸F]flouro-L-arabinofuranosyl)-5-methyluracil (-[¹⁸F]FMAU)(Scher, B.; et al. Eur J Nucl Med Mol Imaging 2007, 34, 45-53; Rinnab, L.; et al. BJU Int 2007, 100, 786,793; Reske, S.N.; et al. J Nucl Med 2006, 47, 1249-1254; Zophel, K.; Kotzerke, J. Eur J Nucl Med Mol Imaging 2004, 31, 756-759; Vees, H.; et al. BJU Int 2007, 99, 1415-1420; Larson, S. M.; et al. J Nucl Med 2004, 45, 366-373; Schuster, D.M.; et al. J Nucl Med 2007, 48, 56-63; Tehrani, O.S.; et al. J Nucl Med 2007, 48, 1436-1441). Each operates by a different mechanism and has certain advantages, e.g., low urinary excretion for [¹¹C]choline, and disadvantages, such as the short physical half-life of positron-emitting radionuclides.

It is well known that tumors may express unique proteins associated with their malignant phenotype or may over-express normal constituent proteins in greater number than normal cells. The expression of distinct proteins on the surface of tumor cells offers the opportunity to diagnose and characterize disease by probing the phenotypic identity and biochemical composition and activity of the tumor. Radioactive molecules that selectively bind to specific tumor cell surface proteins provide an attractive route for imaging and treating tumors under non-invasive conditions. A promising new series of low molecular weight imaging agents targets the prostate-specific membrane antigen (PSMA) (Mease R.C. et al. Clin Cancer Res. 2008, 14, 3036-3043; Foss, C.A.; et al. Clin Cancer Res 2005, 11, 4022-4028; Pomper, M.G.; et al. Mol Imaging 2002, 1, 96-101; Zhou, J.; etr al. Nat Rev Drug Discov 2005, 4, 1015-1026; WO 2013/022797).

PSMA is a trans-membrane, 750 amino acid type II glycoprotein that has abundant and restricted expression on the surface of PCa, particularly in androgen-independent, advanced and metastatic disease (Schulke, N.; et al. Proc Natl Acad Sci U S A 2003, 100, 12590-12595). The latter is important since almost all PCa become androgen independent over the time. PSMA possesses the criteria of a promising target for therapy, i.e., abundant and restricted (to prostate) expression at all stages of the disease, presentation at the cell surface but not shed into the circulation and association with enzymatic or signaling activity (Schulke, N.; et al. Proc. Natl. Acad. Sci. USA 2003, 100, 12590-12595). The PSMA gene is located on the short arm of chromosome 11 and functions both as a folate hydrolase and neuropeptidase. It has neuropeptidase function that is equivalent to glutamate carboxypeptidase II (GCPII), which is referred to as the "brain PSMA", and may modulate glutamatergic transmission by cleaving *N-*acetylaspartylglutamate (NAAG) to N-acetylaspartate (NAA) and glutamate (Nan, F.; et al. J Med Chem 2000, 43, 772-774). There are up to 10⁶ PSMA molecules per cancer cell, further suggesting it as an ideal target for imaging and therapy with radionuclide-based techniques (Tasch, J.; et al. Crit Rev Immunol 2001, 21, 249-261).

The radio-immunoconjugate of the anti-PSMA monoclonal antibody (mAb) 7E11, known as the PROSTASCINT® scan, is currently being used to diagnose prostate cancer metastasis and recurrence. However, this agent tends to produce images that are challenging to interpret (Lange, P.H. PROSTASCINT scan for staging prostate cancer. Urology 2001, 57, 402-406*;* Haseman, M.K.; et al. Cancer Biother Radiopharm 2000, 15, 131-140; Rosenthal, S.A.; et al. Tech Urol 2001, 7, 27-37). More recently, monoclonal antibodies have been developed that bind to the extracellular domain of PSMA and have been radiolabeled and shown to accumulate in PSMA-positive prostate tumor models in animals. However, diagnosis and tumor detection using monoclonal antibodies has been limited by the low permeability of the monoclonal antibody in solid tumors.

The selective targeting of cancer cells with radiopharmaceuticals, either for imaging or therapeutic purposes is challenging. A variety of radionuclides are known to be useful for radio-imaging or cancer radiotherapy, including ¹¹¹In, ⁹⁰Y, ⁶⁸Ga, ¹⁷⁷Lu, ^{99m}Tc, ¹²³I and ¹³¹I. Recently it has been shown that some compounds containing a glutamate-urea-glutamate (GUG) or a glutamate-urea-lysine (GUL) recognition element linked to a radionuclide-ligand conjugate exhibit high affinity for PSMA.

New agents that will enable rapid visualization of prostate cancer and specific targeting to allow radiotherapy present are needed.

Thus, the object of the present invention is to develop ligands that interact with PSMA and carry appropriate radionuclides which provide a promising and novel targeting option for the detection, treatment and management of prostate cancer.

### BRIEF DESCRIPTION OF THE FIGURES

Fig. 1: ¹⁸F-Labelling of macromolecules
Fig. 2: ¹⁸F-Fluorination via prosthetic groups
Fig. 3: ¹⁸F-prosthetic groups for peptides
Fig. 4: ¹⁸F-Labelled Prosthetic Groups using "Click Chemistry"
Fig. 5: Formation of [¹⁸F]aryltrifluoroboronates and fluoride sensors
Fig. 6: Complexes of ¹⁸F-Fluorides
Fig. 7: ¹⁸F-Labelling of RGD peptides

### SUMMARY OF THE INVENTION

The solution of said object is achieved by providing the embodiments characterized in the claims.

The inventors found new compounds which are useful radiopharmaceuticals and their use in nuclear medicine as tracers, imaging agents and for the treatment of various disease states of prostate cancer.

The novel imaging agents with structural modifications in the linker region have improved tumor targeting properties and pharmacokinetics. The pharmacophore presents three carboxylic groups able to interact with the respective side chains of PSMA and an oxygen as part of zinc complexation in the active center. Besides these obligatory interactions, the inventors were able to optimize the lipophilic interactions in the linker region.

### DETAILED DESCRIPTION OF THE INVENTION

The present invention relates to radiopharmaceuticals and their use in nuclear medicine as tracers, imaging agents and for the treatment of various disease states of prostate cancer.

Thus, the present invention concerns compounds that are represented by the general Formulae (Ia) or (Ib): 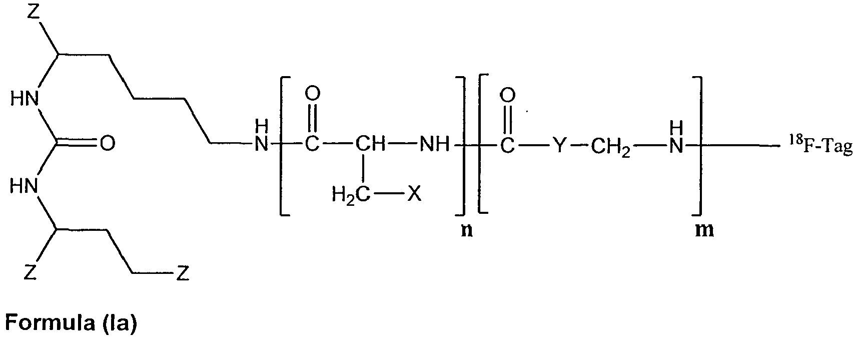 or 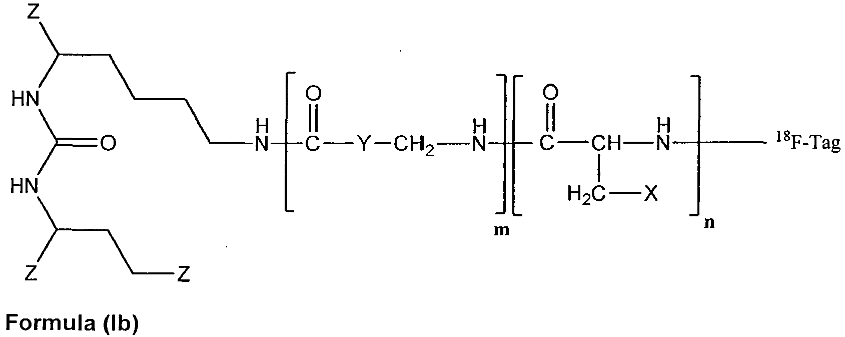 with:

| | |
|---|---|
| n: | 0,1 |
| m: | 0,1,2,3,4 with the proviso that m+n > 0 |
| Z: | -CO₂H, -SO₂H, -SO₃H, -SO₄H, -PO₂H, -PO₃H, -PO₄H₂ |
| X: | Naphthyl, Phenyl, Biphenyl, Indolyl (=2,3-benzopyrrolyl), Benzothiazolyl |
| Y: | Aryl, Alkylaryl, Cyclopentyl, Cyclohexyl, Cycloheptyl |
| ¹⁸F-Tag: | |
| | 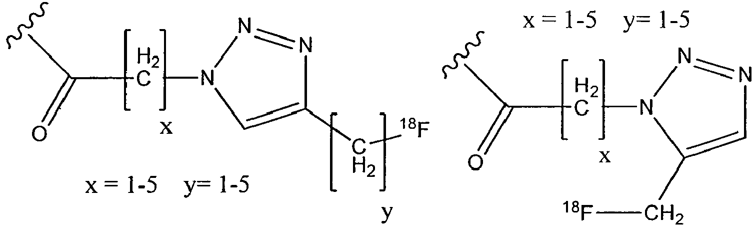 |
| | 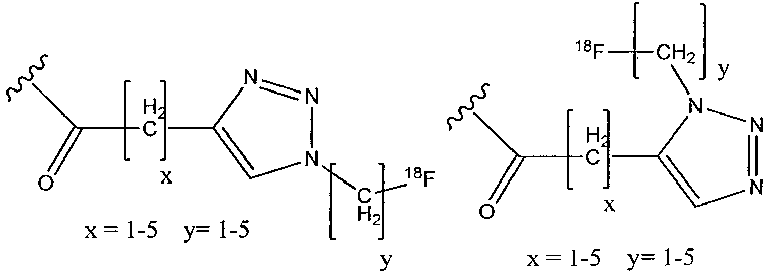 |
| | 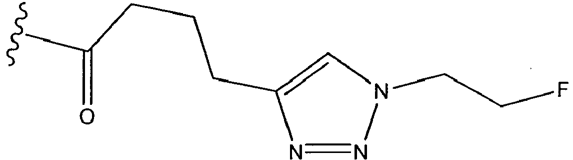 |
| | 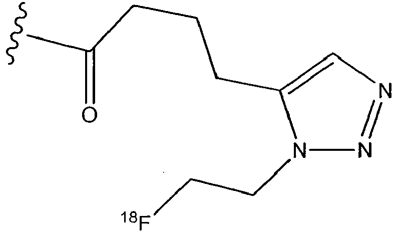 |
| | 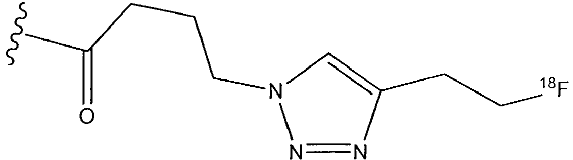 |
| | 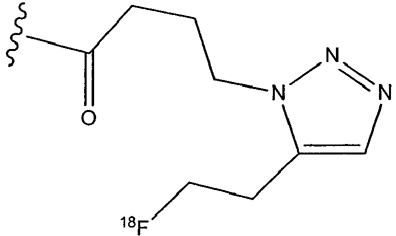 |
| | 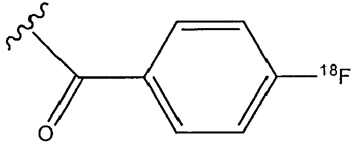 |
| | 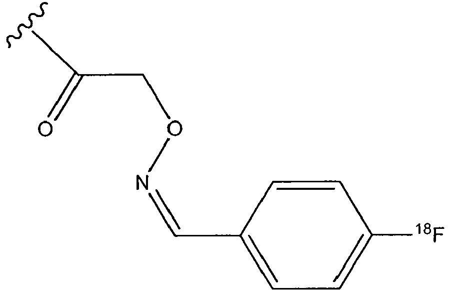 |
| | 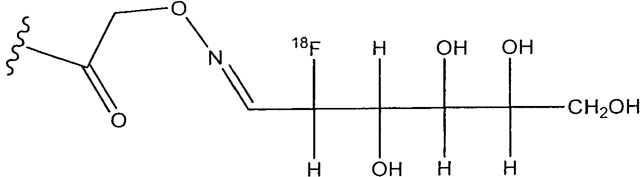 |
| | 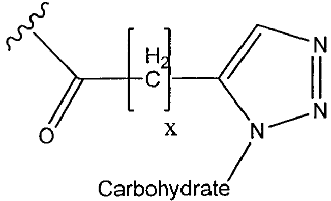 |
| | 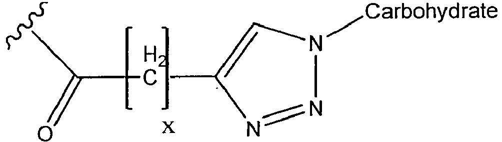 |
| | With: |
| | x = 1-5 |
| | Carboydrate : |
| | 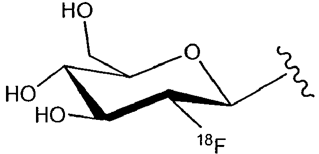 |
| | 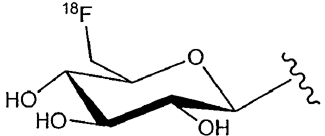 |
| | 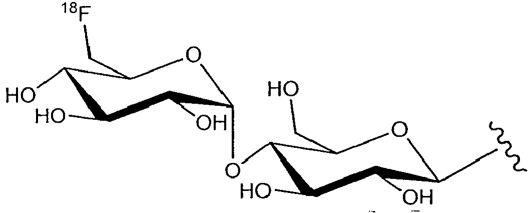 |
| | 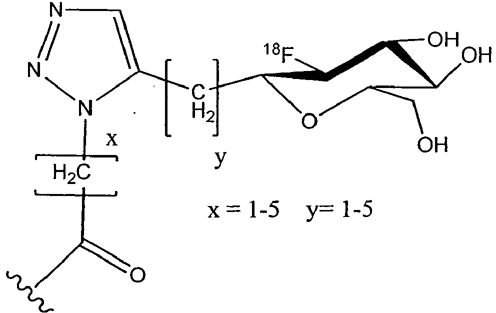 |
| | 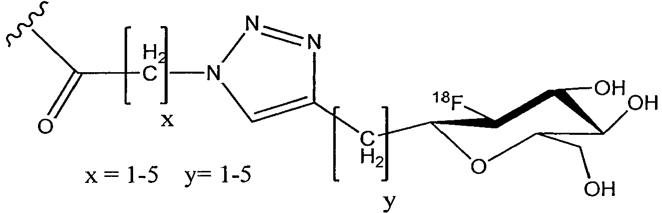 |
| | 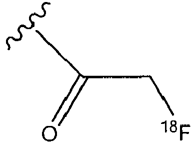 |
| | 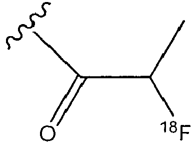 |
| | 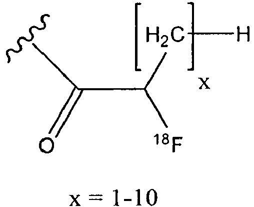 |
| | 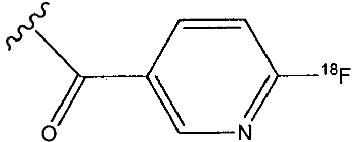 |
| | 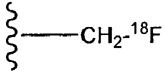 |
| | 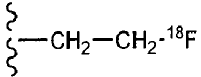 |
| | 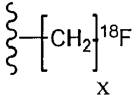 |
| | x = 1-10 |
| | 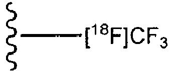 |
| | 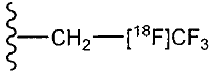 |
| | 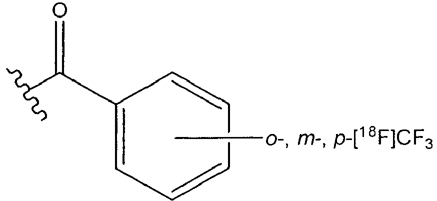 |
| | 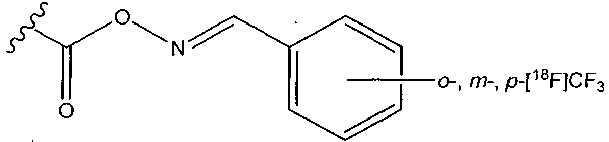 |
| | 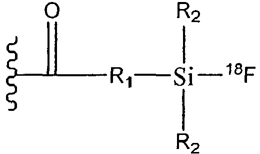 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 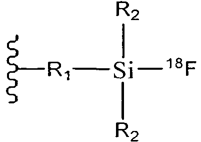 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 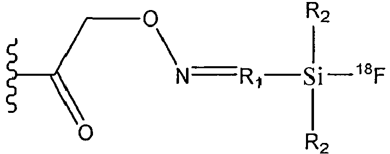 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 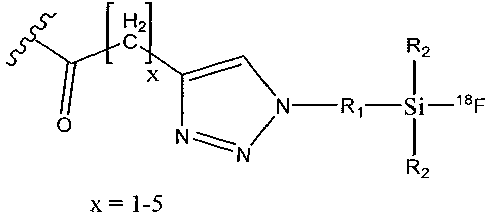 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 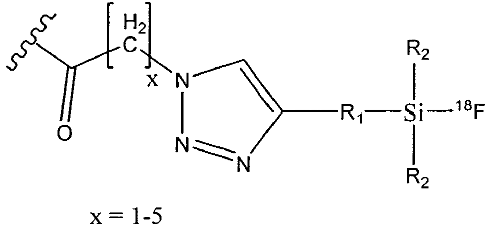 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 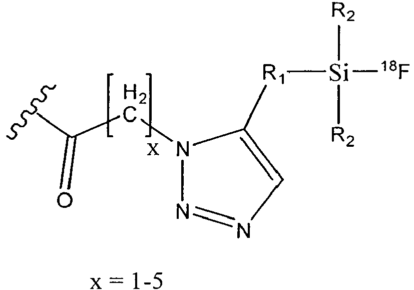 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 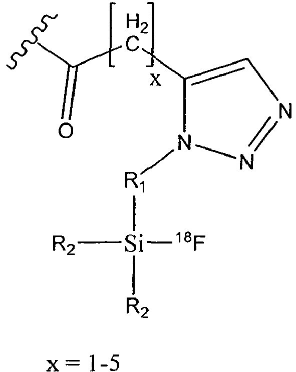 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |

If not stated otherwise, in the present invention the "alkyl" residue (preferably: C₁ to C₁₀) can be linear or branched, unsubstituted or substituted. Preferred alkyl residues are methyl, ethyl, n-propyl, iso-propyl, n-butyl, tert-butyl, n-pentanyl, n-hexanyl. The same also applies to the corresponding cycloalkyl compounds having preferably 3 to 10 carbon atoms.

"Aryl" refers to an aromatic monocyclic or polycyclic ring system having 6 to 14 carbon atoms, preferably 6 to 10 carbon atoms. The aryl group can be substituted, where appropriate, with one or several ring substituents, like alkyl groups. Preferred aryl groups are phenyl, benzyl or naphthyl.

Although it is preferred that the Z-Group is -CO₂H it may be easily replaced with biosteric replacements such as -SO₂H, -SO₃H, -SO₄H, -PO₂H, -PO₃H, -PO₄H₂, see e.g. "The Practice of Medicinal Chemistry" (Academic Press New York, 1996), page 203.

Within the meaning of the invention, all residues are considered combinable unless stated otherwise in the definition of the residues. All conceivable subgroupings thereof are considered to be disclosed.

The ¹⁸F-Tags of the above Table comprising triazoles exist in two isomeric forms which belong both to the invention and are illustrated by the given formulas

In a preferred embodiment, the motif specifically binding to cell membranes of neoplastic cells is a motif specifically binding to cell membranes of cancerous cells, preferably wherein said motif comprises a prostate-specific membrane antigen (PSMA), in particular wherein said PSMA comprises a glutamate-urea-lysine motif according to the following formula in Scheme 1.

Thus, preferred molecules of the present invention consist of three principle components (Scheme 1): the hydrophilic PSMA binding motif (Glu-Urea-Lys = Glu-NH-CO-NH-Lys), a variable linker and the ¹⁸F-Tag.

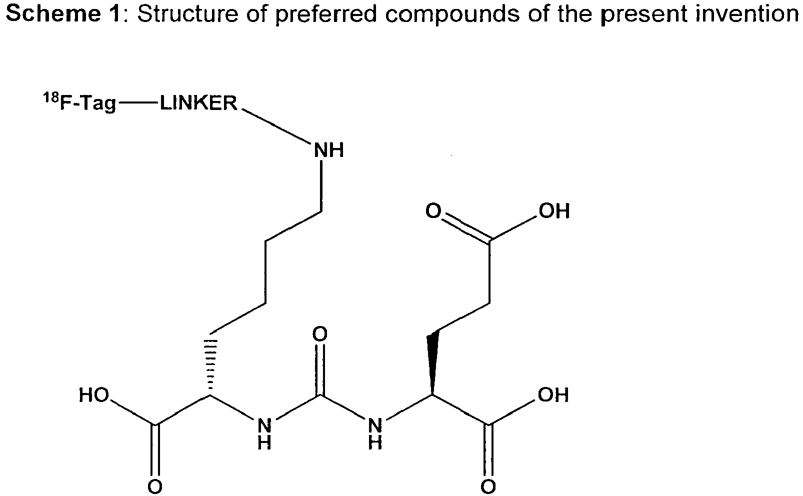

The different preferred linkers are shown below, wherein R=Glu-urea-Lys and R'= ¹⁸F-Tag, as shown above. 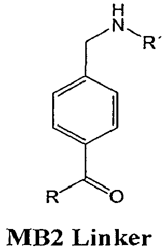 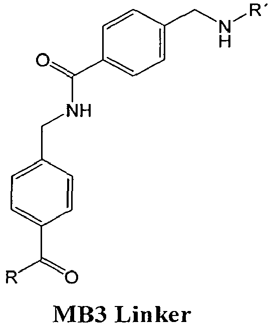 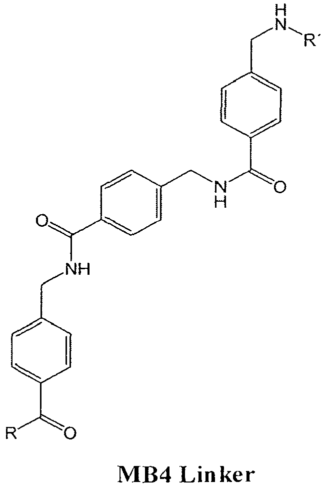 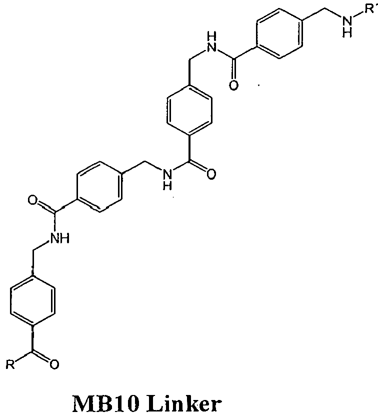 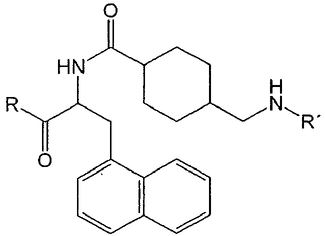 or

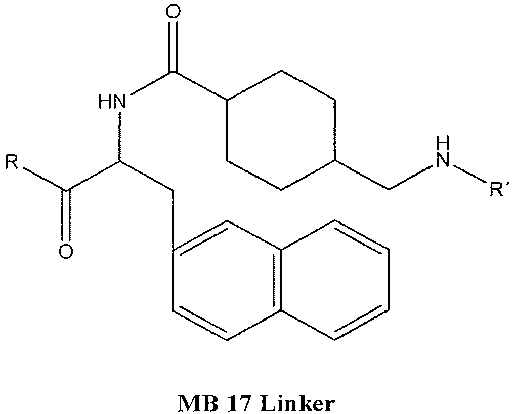

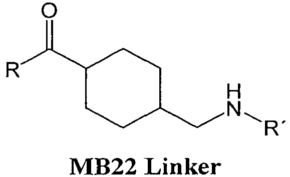

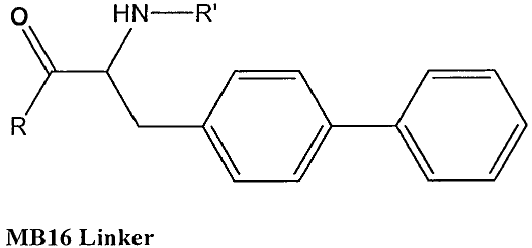

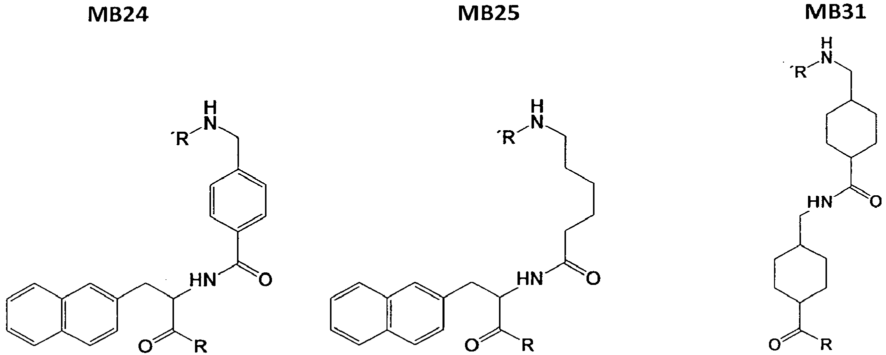

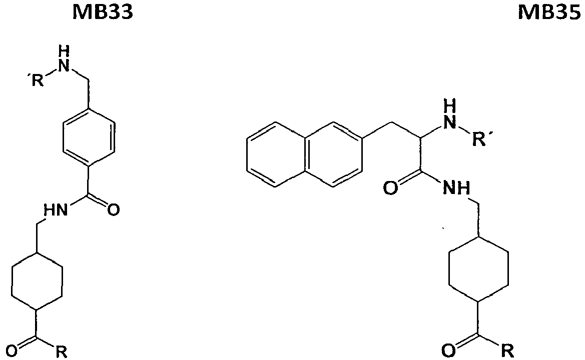

Preferred compounds of the present invention are shown below (with X, Y and Z as defined above in the Table in connection with Formula I(a) and I(b))

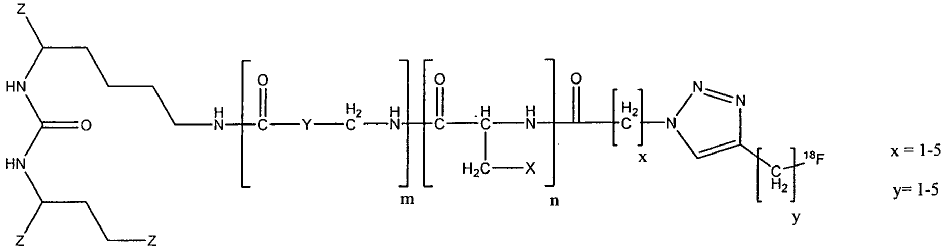

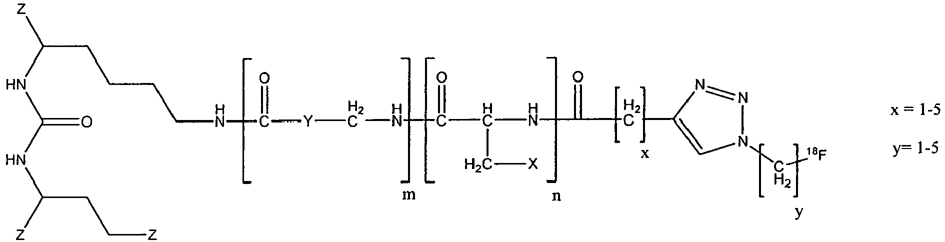

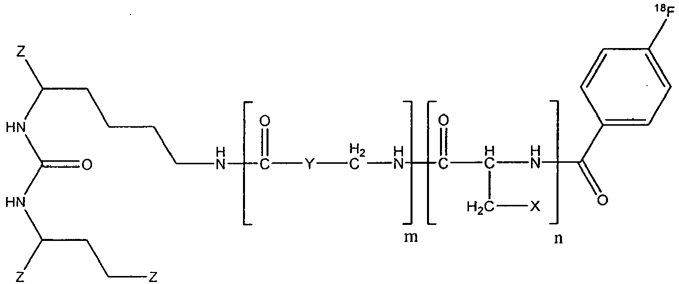

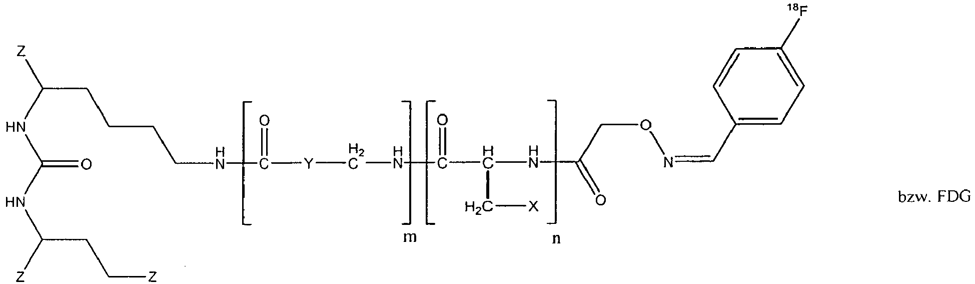

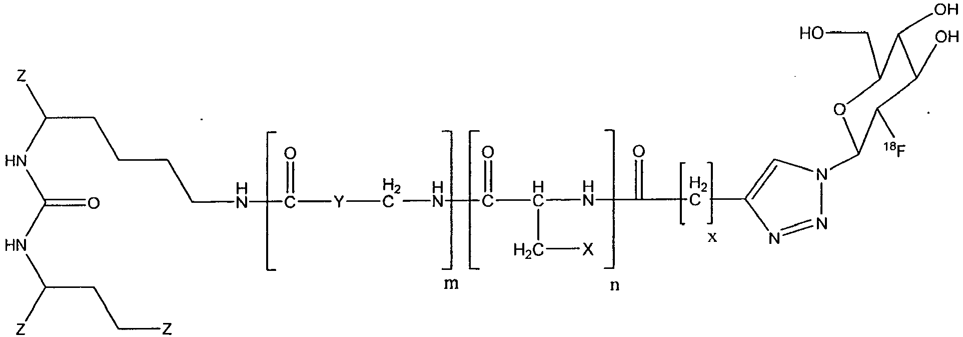

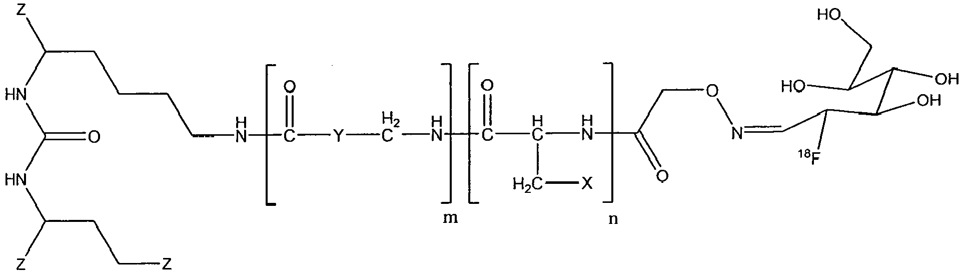

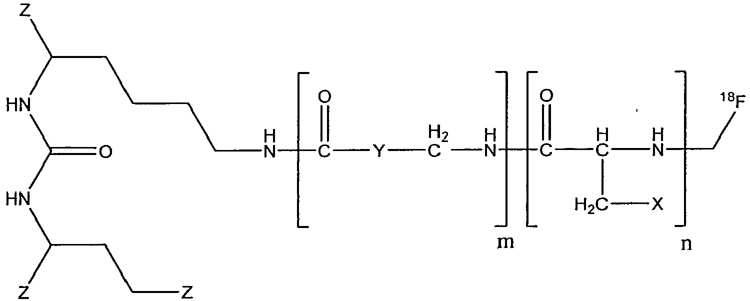

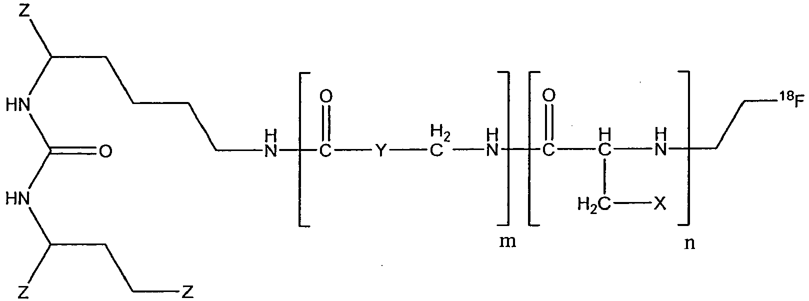

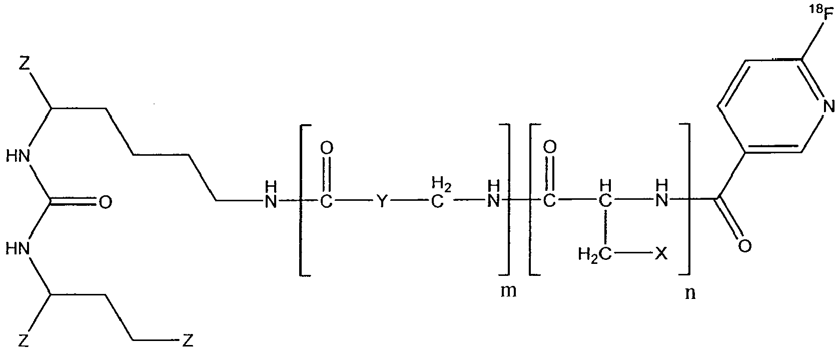

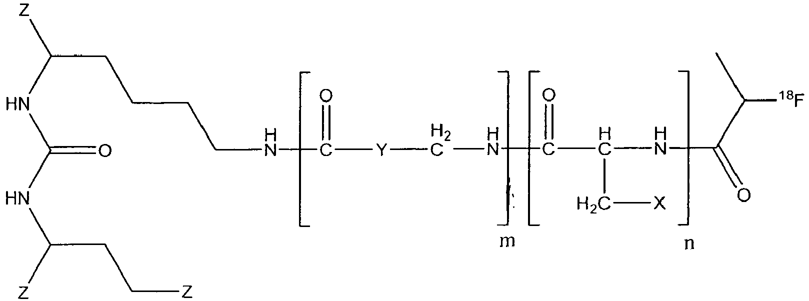

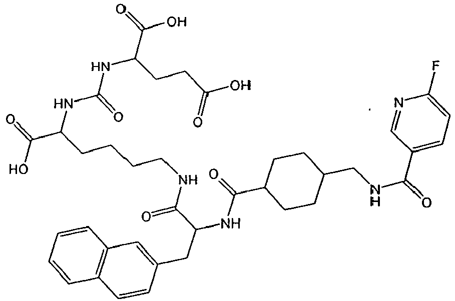

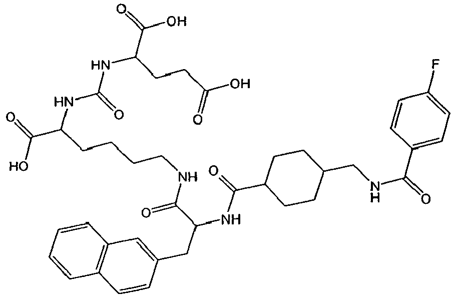

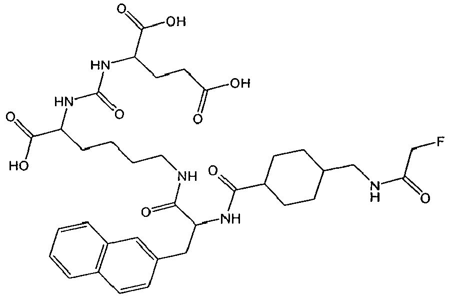

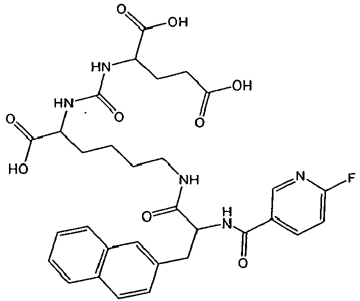

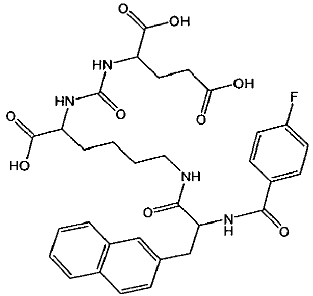

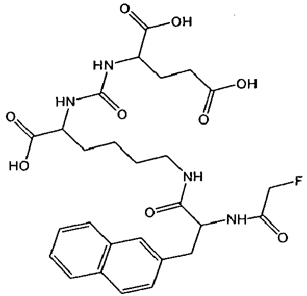

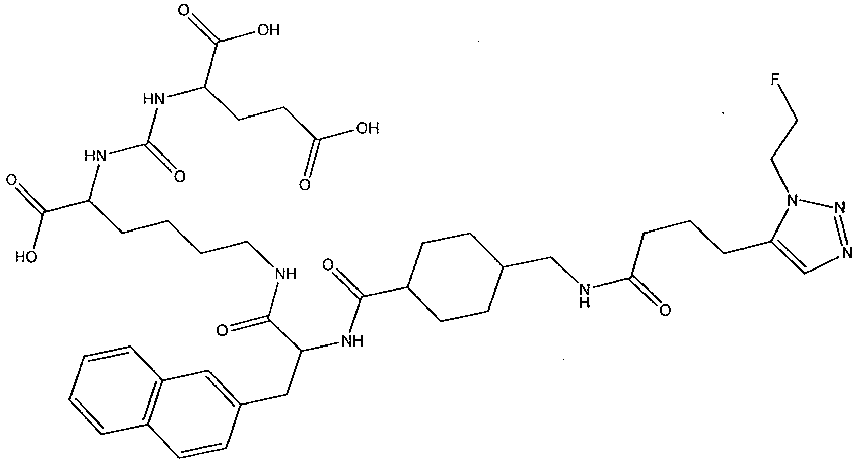

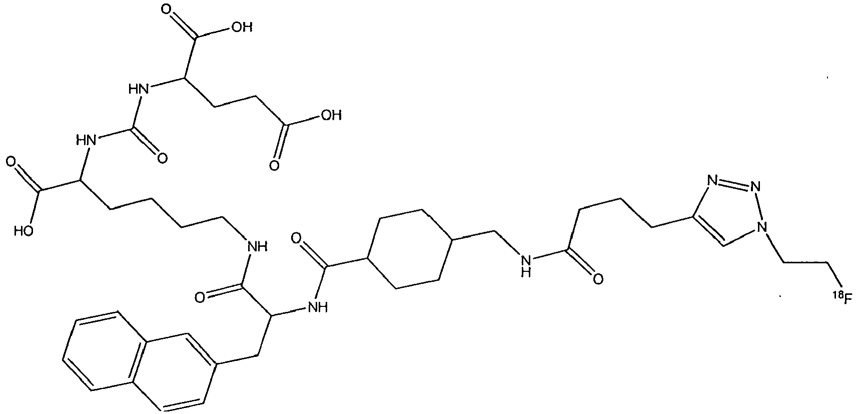

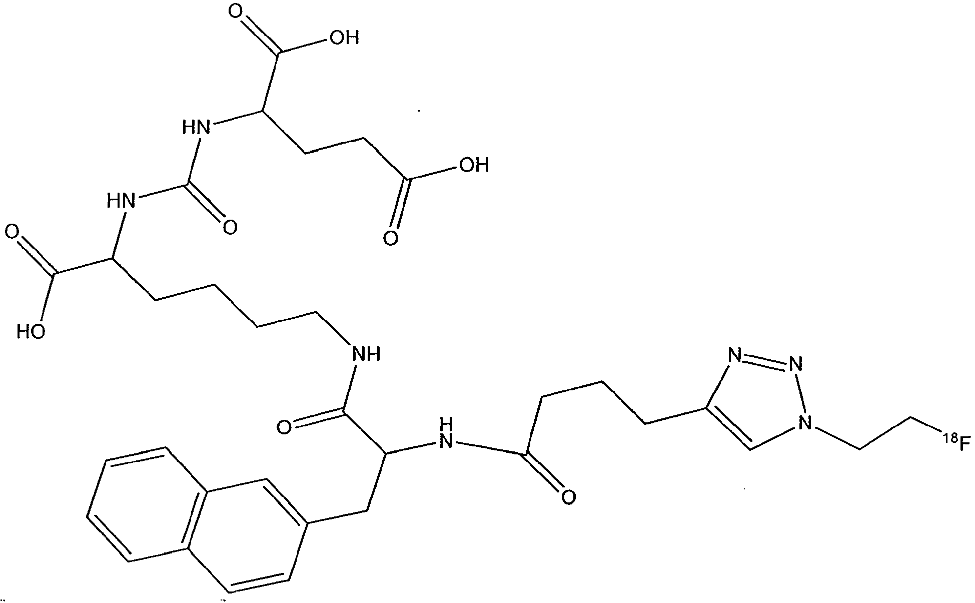

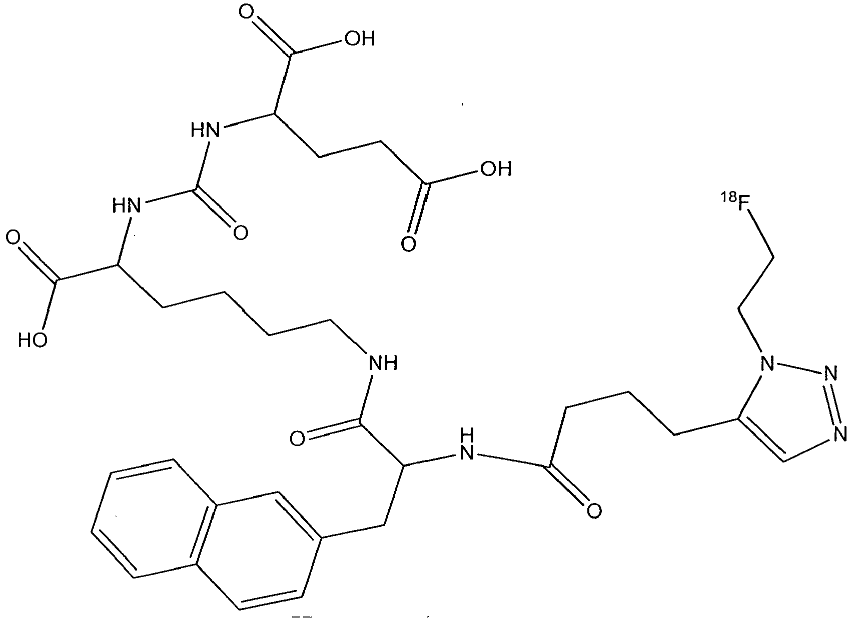

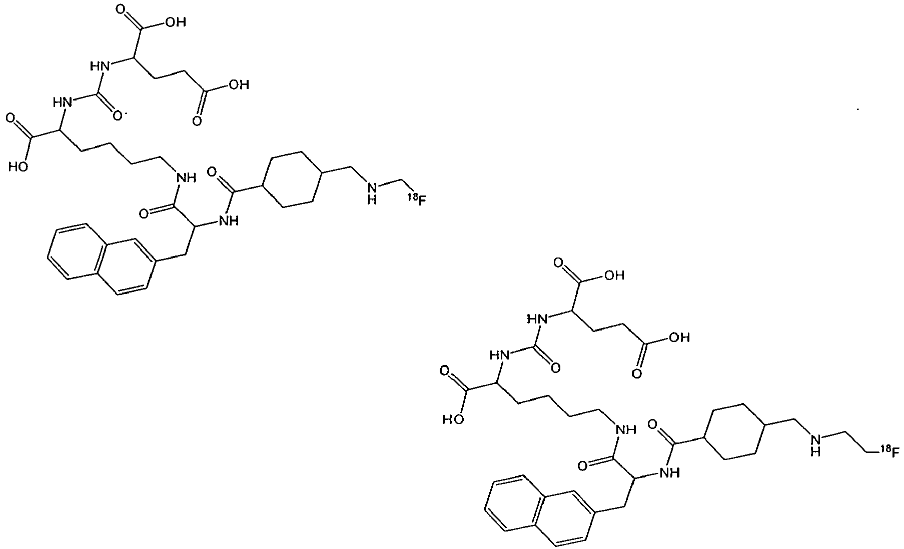

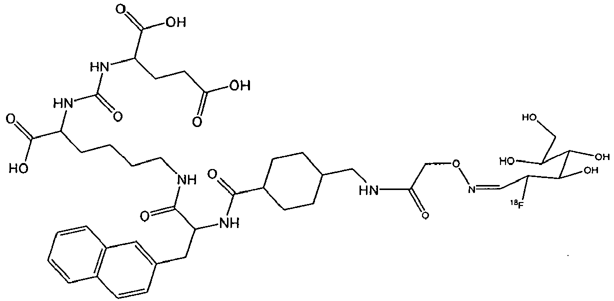

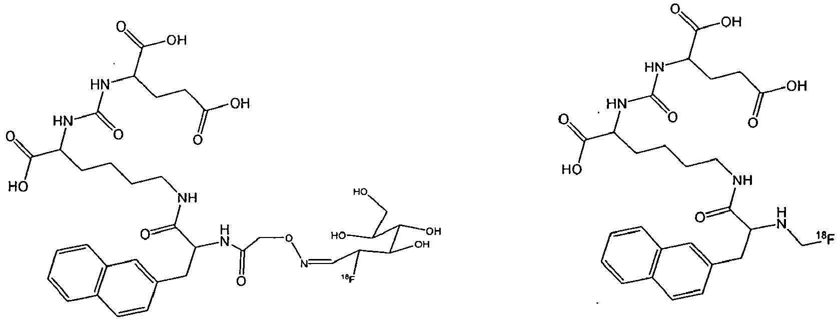

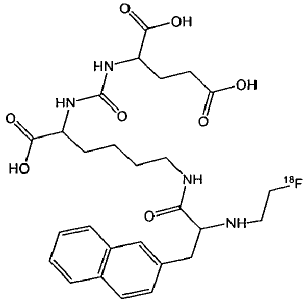

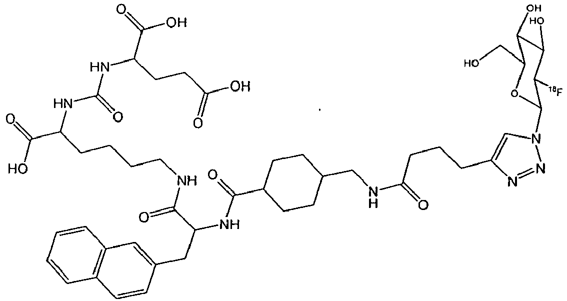

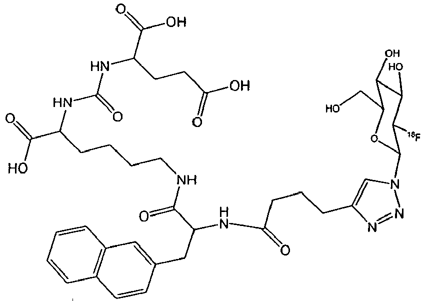

The invention also relates to pharmaceutically acceptable salts of the compounds of general formula (Ia) and/or (Ib). The invention also relates to solvates of the compounds, including the salts as well as the active metabolites thereof and, where appropriate, the tautomers thereof according to general formula (Ia) and/or (Ib) including prodrug formulations.

A "pharmaceutically acceptable salt" is a pharmaceutically acceptable, organic or inorganic acid or base salt of a compound of the invention. Representative pharmaceutically acceptable salts include, e.g., alkali metal salts, alkali earth salts, ammonium salts, water-soluble and water-insoluble salts, such as the acetate, carbonate, chloride, gluconate, glutamate, lactate, laurate, malate or tartrate.

The term "prodrug" refers to a precursor of a drug that is a compound which upon administration to a patient, must undergo chemical conversion by metabolic processes before becoming an active pharmacological agent. Illustrative prodrugs of compounds in accordance with Formula (Ia) and/or (Ib) are esters and amides, preferably alkyl esters of fatty acid esters. Prodrug formulations here comprise all substances which are formed by simple transformation including hydrolysis, oxidation or reduction either enzymatically, metabolically or in any other way. A suitable prodrug contains e.g. a substance of general formula (Ia) and/or (Ib) bound via an enzymatically cleavable linker (e.g. carbamate, phosphate, N-glycoside or a disulfide group) to a dissolution-improving substance (e.g. tetraethylene glycol, saccharides, formic acids or glucuronic acid, *etc.*). Such a prodrug of a compound according to the invention can be applied to a patient, and this prodrug can be transformed into a substance of general formula (Ia) and/or (Ib) so as to obtain the desired pharmacological effect.

Some compounds of Formula (Ia) and/or (Ib) are encompassed in form of the racemates, their enantiomers and optionally in form of their diastereomers and all possible mixtures thereof.

According to the invention all chiral C-atoms shall have D- and/or L-configuration; also combinations within one compound shall be possible, i.e. some of the chiral C-atoms may be D- and others may be L-configuration.

The obtained compounds can be optionally separated by known methods (e.g. Allinger, N.L. und Elliel E.L. in "Topics in Stereochemistry" Vol. 6, Wiley Interscience, 1971*)* in their enantiomers and/or diasteromers. One possible method of enantiomeric separation is the use of chromatography.

The invention also relates to pharmaceutical preparations which contain a therapeutically effective amount of the active ingredients (compound according to the invention of formula (Ia) or (Ib) together with organic or inorganic solid or liquid, pharmaceutically acceptable carriers which are suited for the intended administration and which interact with the active ingredients without drawbacks.

The phrase "pharmaceutically acceptable" is employed herein to refer to those compounds, material, compositions, and/or dosage forms which are, within the scope of sound medical judgment, suitable for use in contact with the tissues of a patient without excessive toxicity, irritation, allergic response, or other problem or complication, commensurate with a reasonable benefit/risk ratio.

A "patient" includes an animal, such as a human, monkey, cow, horse, cat or dog. The animal can be a mammal such as a non-primate and a primate (e.g., monkey and human). In one embodiment, a patient is a human being.

In general, the Formula (Ia) or (Ib) compound or pharmaceutical compositions thereof, may be administered orally or via a parenteral route, usually injection or infusion.

A "parenteral administration route" means modes of administration other than enteral and topical administration, usually by injection, and includes, without limitation, intravenous, intramusclular, intraarterial, intrathecal, intracapsular, intraorbital, intracardiac, intradermal, intraperitoneal, transtracheal, subcutaneous, subcuticular, intraarticluare, subcapsular, subarachnoid, intraspinal and intrasternal injection and infusion.

The dosage of the compounds according to the invention is determined by the physician on the basis of the patient-specific parameters, such as age, weight, sex, severity of the disease, *etc.* The dosage is preferably from 0.00001 mg/kg to 100 mg/kg body weight, preferably from 0.001 to 50 mg/kg body weight and most preferably from 0.01 to 10 mg/kg body weight.

Corresponding to the kind of administration, the medicament is suitably formulated, e.g. in the form of solutions or suspensions, simple tablets or dragees, hard or soft gelatine capsules, suppositories, ovules, preparations for injection, which are prepared according to common galenic methods.

The compounds according to the invention can be formulated, where appropriate, together with further active substances and with excipients and carriers common in pharmaceutical compositions, e.g. - depending on the preparation to be produced - talcum, gum arabic, lactose, starch, magnesium stearate, cocoa butter, aqueous and non-aqueous carriers, fatty bodies of animal or vegetable origin, paraffin derivatives, glycols (in particular polyethylene glycol), various plasticizers, dispersants or emulsifiers, pharmaceutically compatible gases (e.g. air, oxygen, carbon dioxide, *etc.*), preservatives.

In order to produce liquid preparations, additives, such as sodium chloride solution, ethanol, sorbitol, glycerine, olive oil, almond oil, propylene glycol or ethylene glycol, can be used.

When solutions for infusion or injection are used, they are preferably aqueous solutions or suspensions, it being possible to produce them prior to use, e.g. from lyophilized preparations which contain the active substance as such or together with a carrier, such as mannitol, lactose, glucose, albumin and the like. The ready made solutions are sterilized and, where appropriate, mixed with excipients, e.g. with preservatives, stabilizers, emulsifiers, solubilizers, buffers and/or salts for regulating the osmotic pressure. The sterilization can be obtained by sterile filtration using filters having a small pore size according to which the composition can be lyophilized, where appropriate. Small amounts of antibiotics can also be added to ensure the maintenance of sterility.

The phrases "effective amount" or "therapeutically-effective amount" as used herein means that amount of a compound, material, or composition comprising a compound of the invention, or other active ingredient which is effective for producing some desired therapeutic effect in at least a sub-population of cells in an animal at a reasonable benefit/risk ratio applicable to any medical treatment. A therapeutically effective amount with respect to a compound of the invention means that amount of therapeutic agent alone, or in combination with other therapies, that provides a therapeutic benefit in the treatment of prevention of a disease. Used in connection with a compound of the invention, the term can encompass an amount that improves overall therapy, reduces or avoids symptoms or causes of disease, or enhances the therapeutic efficacy of or synergies with another therapeutic agent.

As used herein, the terms "treating" or "treatment" is intended to encompass also diagnosis, prophylaxis, prevention, therapy and cure.

The terms "prevent", "preventing," and "prevention" refer to the prevention of the onset, recurrence, or spread of the disease in a patient resulting from the administration of a prophylactic or therapeutic agent.

As noted above, compounds according Formula (Ia) or (Ib) are suitable for use as radio-imaging agents or as therapeutics for the treatment of rapidly proliferating cells, for example, PSMA expressing prostate cancer cells. According to the present invention they are called "radiopharmaceuticals".

Preferred imaging methods are positron emission tomography (PET) or single photon emission computed tomography (SPECT).

Accordingly, in one embodiment, a pharmaceutical composition is provided including a compound of Formula (Ia) or Formula (Ib), a salt, solvate, stereoisomer, or tautomer thereof, and a pharmaceutically acceptable carrier. Accordingly, a pharmaceutical composition is provided, which is suitable for in vivo imaging and radiotherapy. Suitable pharmaceutical compositions may contain the compound of Formula (Ia) and/or (Ib) in an amount sufficient for imaging, together with a pharmaceutically acceptable radiological vehicle. The radiological vehicle should be suitable for injection or aspiration, such as human serum albumin; aqueous buffer solutions, e.g., tris(hydromethyl) aminomethane (and its salts), phosphate, citrate, bicarbonate, etc; sterile water physiological saline; and balanced ionic solutions containing chloride and or dicarbonate salts or normal blood plasma cautions such as calcium potassium, sodium and magnesium.

The concentration of the imaging agent or the therapeutic agent in the radiological vehicle should be sufficient to provide satisfactory imaging. For example, when using an aqueous solution, the dosage is about 1.0 to 100 millicuries. The actual dose administered to a patient for imaging or therapeutic purposes, however, is determined by the physician administering treatment. The imaging agent or therapeutic agent should be administered so as to remain in the patient for about 1 hour to 10 days, although both longer and shorter time periods are acceptable. Therefore, convenient ampoules containing 1 to 10 mL of aqueous solution may be prepared.

Imaging may be carried out in the normal manner, for example by injecting a sufficient amount of the imaging composition to provide adequate imaging and then scanning with a suitable imaging or scanning machine, such as a tomograph or gamma camera. In certain embodiments, a method of imaging a region in a patient includes the steps of: (i) administering to a patient a diagnostically effective amount of a compound complexed with a radionuclide; exposing a region of the patient to the scanning device; and (ii) obtaining an image of the region of the patient. In certain embodiments of the region imaged is the head or thorax. In other embodiments, the compounds and complexes of Formula I(a) and/or (Ib) target the PSMA protein.

Thus, in some embodiments, a method of imaging tissue such as spleen tissue, kidney tissue, or PSMA-expressing tumor tissue is provided including contacting the tissue with a complex synthesized by contacting a radionuclide and a Formula (Ia) and/or Formula (Ib) compound.

The amount of the compound of the present invention, or its salt, solvate, stereoisomer, or tautomer that is administered to a patient depends on several physiological factors that are routinely used by the physician, including the nature of imaging to be carried out, tissue to be targeted for imaging or therapy and the body weight and medical history of the patient to be imaged or treated using a radiopharmaceutical.

Accordingly in another aspect, the invention provides a method for treating a patient by administering to a patient a therapeutically effective amount of a Formula (Ia) and/or (Ib) compound complexed to a radionuclide, or a pharmaceutically acceptable salt or solvate of the complex to treat a patient suffering from a cell proliferative disease or disorder. Specifically, the cell proliferative disease or disorder to be treated or imaged using a compound, pharmaceutical composition or radiopharmaceutical in accordance with this invention is a cancer, for example, prostate cancer and/or prostate cancer metastasis in e.g. lung, liver, kidney, bones, brain, spinal cord, bladder, etc.

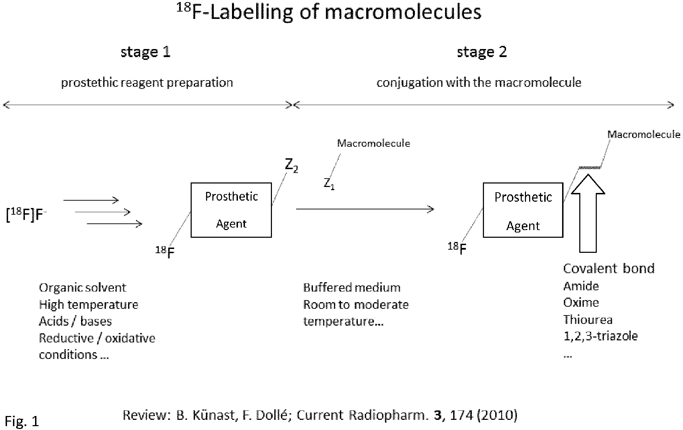

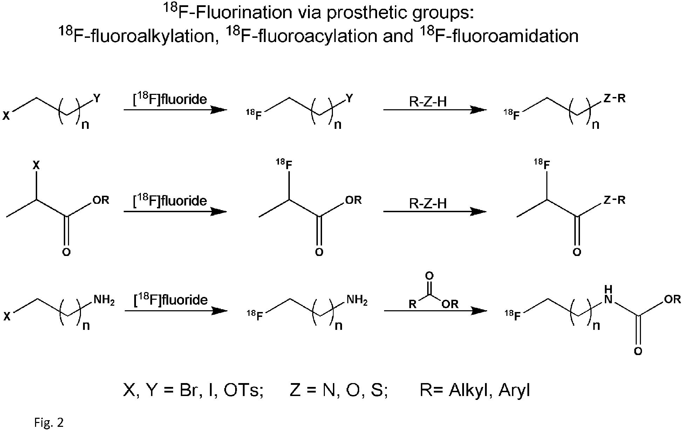

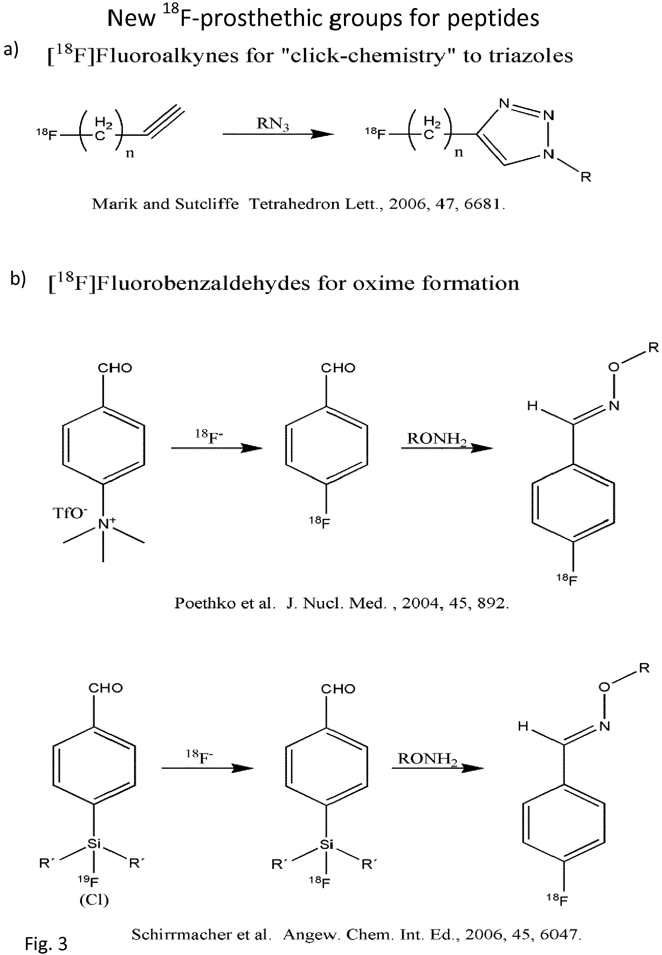

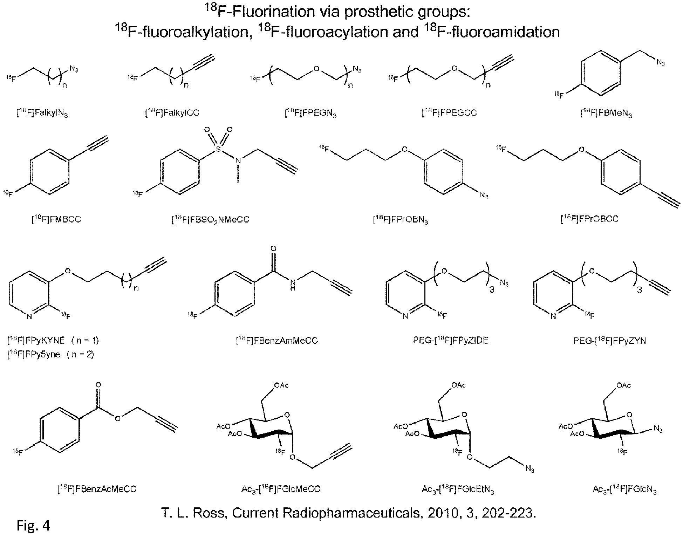

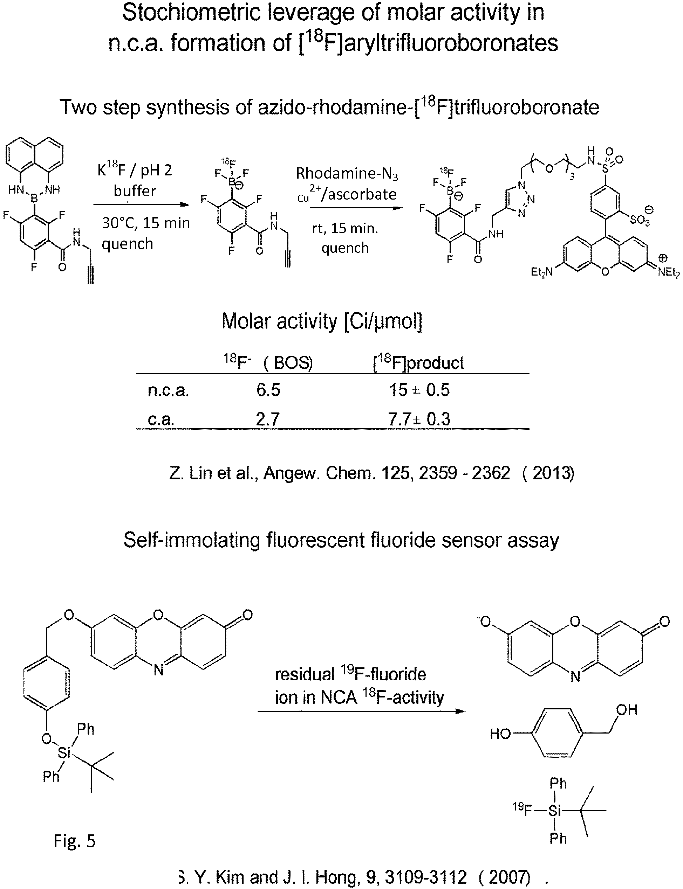

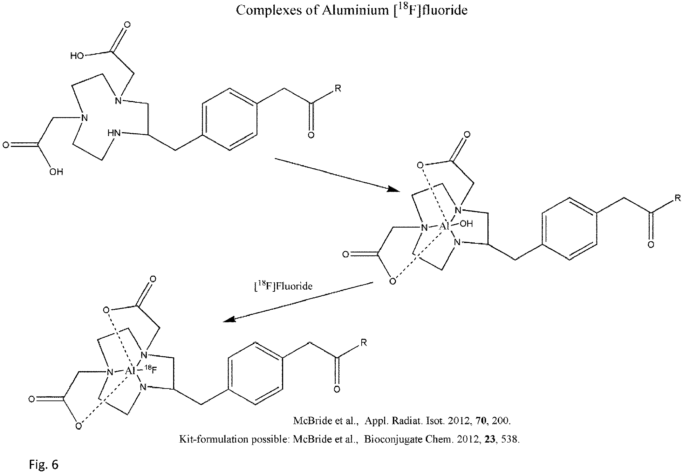

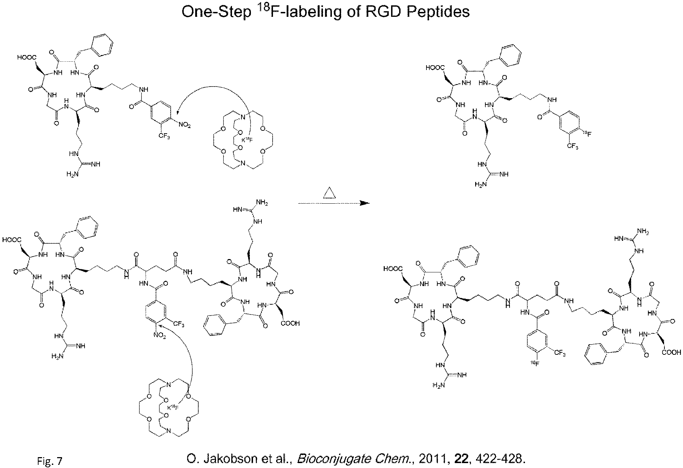

The synthesis of the compounds of the present invention is carried out according to methods well known in the prior art (e.g. Hugenberg et al., J. of Medicinal Chemistry, 2013, 56, pp. 6858-6870). General methods for ¹⁸F-labelling of various macromolecules are shown in Fig. 1-7. In addition, reference is made to Schubiger et al., PET Chemistry:

The Driving Force in Molecular Imaging, Ernst Schering Research Foundation, Workshop 62, Springer Verlag, ISSN 0947-6075; Ross et al., Current Radiopharmaceuticals, 2010, 3, 202-223; Kühnast et al., Current Radiopharm 3, 2010, 174; Bernard-Gauthier et al., BioMed Research International, 2014, 1; Maschauer and Prante, BioMed Research International, 2014, 1; Olberg et al., J. Med. Chem., 2010, 53, 1732; Rostovtsev et al., Angew. Chem., 2002, 114, 2708; Smith and Greaney, Org. Lett., 2013, 15, 4826. Thus, a person skilled in the art would be able to choose the right ¹⁸F-labelling depending on the starting molecule. The synthesis of the specific linker molecules is shown in EP 13004991 to which reference is made.

The synthesized compounds are chemically characterized by RP-HPLC, MS, and/or NMR.

The novel ¹⁸F-tagged imaging agents with structural modifications in the linker region have improved tumor targeting properties and pharmacokinetics. The pharmacophore presents three carboxylic group able to interact with the respective side chains of PSMA and an oxygen as part of zinc complexation in the active center. Besides these obligatory interactions, the inventors were able to optimize the lipophilic interactions in the linker region.

The preclinical evaluation includes *in vitro* assays (affinity, internalization) and *in vivo* experiments (µPET screening and organ distribution).

The compounds of the present invention are better than known reference compounds with regard to kidney clearance and enrichment in the tumor. The binding affinity of PSMA inhibitors of the present invention can be influenced by linker modifications. Two cyclic motives and at least one aromatic moiety in the linker region of the substance seem to be preferable and resulted in the high affinity compounds MB4 and MB17. In this regard, a very promising compound is MB17.

Thus, the compounds of the present invention represent novel PSMA-targeting probes with optimal characteristics which was also confirmed by organ distribution and small animal PET imaging. The compounds of the present invention show a high PSMA-specific tumor uptake. In addition, they are characterized by an early enrichment in the bladder and also the maximum kidney uptake. With regard to therapeutic use, this gives clear clinical advantages for the compounds of the present invention compared to other PSMA-inhibitors. In the PET diagrams the compounds of the present invention, in particular MB17, show a rapid background clearance as well as a substantial reduction of the enrichment in the kidney after 2 hours while it is further accumulated and retained in the PSMA-expressing tumor.

The below example explains the invention in more detail but are not construed to limit the invention in any way to the exemplified embodiments only.

### Examples

### Example 1: Synthesis of ¹⁸F-conjugated inhibitors

The isocyanate of the glutamyl moiety was generated in situ by adding a mixture of 3 mmol of bis(tert-butyl) L-glutamate hydrochloride and 1.5 mL of N-ethyldiisopropylamine (DIPEA) in 200 mL of dry CH2Cl2 to a solution of 1 mmol triphosgene in 10 mL of dry CH2Cl2 at 0 °C over 4 h. After agitation of the reaction mixture for 1 h at 25 °C, 0.5 mmol of the resin-immobilized (2-chloro-tritylresin) ε-allyloxycarbonyl protected lysine in 4 mL DCM was added and reacted for 16 h with gentle agitation. The resin was filtered off and the allyloxy-protecting group was removed using 30 mg tetrakis(triphenyl)palladium(0) and 400 µL morpholine in 4 mL CH2Cl2 for 3 hours.

The following coupling of 0-3 times 4-(Fmoc-aminomethyl)benzoic acid , Fmoc-3-(2-naphthyl)-L-alanine and 0-3 times trans-4-(Fmoc-aminomethyl)cyclohexanecarboxylic acid, respectively, was performed stepwise using 2 mmol of the Fmoc-protected acid, 1.96 mmol of HBTU and 2 mmol of N-ethyldiisopropylamine in a final volume of 4 mL DMF.

The product was cleaved from the resin in a 2 mL mixture consisting of trifluoroacetic acid, triisopropylsilane, and water (95:2.5:2.5). Purification was performed using RP-HPLC and the purified product was analysed by analytical RP-HPLC and MALDI-MS. For the preparation of the non-radioactive reference compounds 50 mg of HBTU/DIPEA (0.98 and 1 Eq.) activated 6-Fluoronicotinic-3-acid was coupled in a final volume of 4 ml DMF and agitated for 1 h at room temperature and the product was the cleaved of the resin as described above.

In some preparations a ¹⁸F-tag reactive moieties (e.g. pent-4-ynoic acid or (Boc-aminooxy)acetic acid) were attached to the terminal amino-group for subsequent ¹⁸F labeling.

### Radiosynthesis:

### Preparation and activation of the [¹⁸F]fluoride

Fluorine-18 was produced by the irradiation of ¹⁸O-enriched water with 16.5 MeV protons using the ¹⁸O(p,n)¹⁸F nuclear reaction. Irradiations were performed with the Scanditronix MC32NI cyclotron at the department of Radiopharmaceutical Chemistry (E030) at the German Cancer Research Center Heidelberg.

After transfer of the irradiated water to an automated system (Trasis All In One 32) the [¹⁸F]F⁻ was separated from the [¹⁸O]H₂O by passing through an previously conditioned (5 ml 1 M K₂CO₃ and 10 ml Water) anion exchange cartridge (Waters Accel™ Plus QMA Cartridge light) and subsequently eluted with a 2 ml acetonitrile containing 20 mg of the aminopolyether Kryptofix® 2.2.2 in 28 µl 1 M K₂CO₃. The mixture was evaporated to dryness at 80° C under a stream of nitrogen. This azeotropic distillation was subsequently repeated two times by adding 1.8 ml of dry acetonitrile for each step. Then the dry residue was dissolved in 2.5 ml of dry acetonitrile and this mixture was used for the labelling reactions

### 6-[¹⁸F]Fluoronicotinic acid tetrafluorophanyl ester

To 10 mg N,N,N-Trimethyl-5-((2,3,5,6-tetrafluorophenoxy)-carbonyl)pyridin-2-aminium trifluoromethanesulfonate 1 ml of *tert*-butanol/Acetonitrile (8:2) containing the dried [¹⁸F]KF-Kryptofix 2.2.2 komplex (2-20 MBq ¹⁸F) was added and the mixture was heated at 40° C. After 10 minutes the mixture was diluted with 3 ml of water and the product loaded on a preconditioned Oasis MCX Plus Sep-Pak (Waters). The cartridge was rinsed with 5 mL of water and the purified 6-[¹⁸F]Fluoronicotinic acid tetrafluorophanyl ester was eluted back to the reaction vessel using 2 mL of water/acetonitrile (7:13).

### Peptide conjugation

The peptide conjugation was accomplished by adding 2 mg of the desired peptide in 1 ml of a 1:1 mixture of DMSO/Buffer (0.2 M phosphate buffer, pH 8.0) to the crude 6-[¹⁸F]Fluoronicotinic acid tetrafluorophanyl ester and subsequent heating of the mixture at 40° C for 10 minutes. The products were analyzed by radio-HPLC and comparison of the retention times with the respective non-radioactive reference compounds.

## Claims

1. A compound of Formula (Ia) or (Ib): 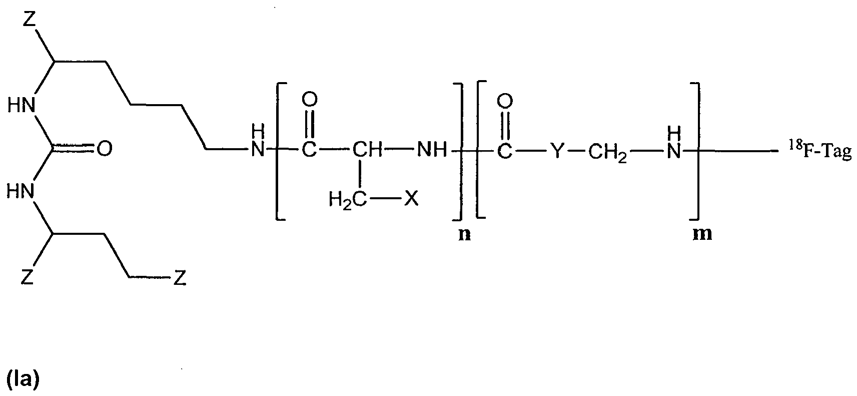 or 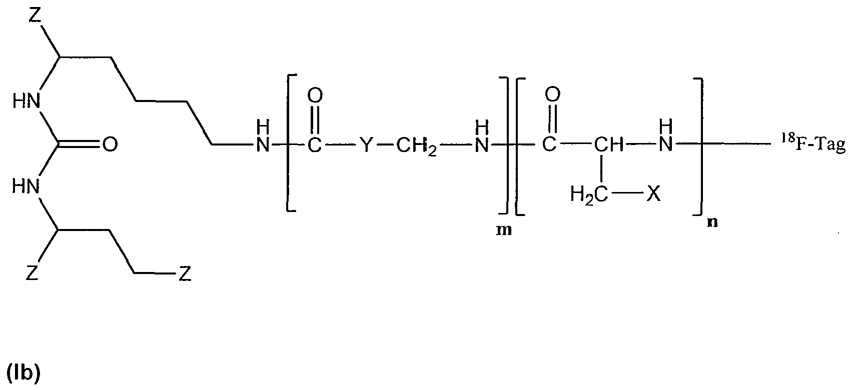 with:
| | |
|---|---|
| n: | 0,1 |
| m: | 0,1,2,3,4 with the proviso that m+n > 0 |
| Z: | -CO₂H, -SO₂H, -SO₃H, -SO₄H, -PO₂H, -PO₃H, -PO₄H₂ |
| X: | Naphthyl, Phenyl, Biphenyl, Indolyl (=2,3-benzopyrrolyl), Benzothiazolyl |
| Y: | Aryl, Alkylaryl, Cyclopentyl, Cyclohexyl, Cycloheptyl |
| ¹⁸F-tag: | 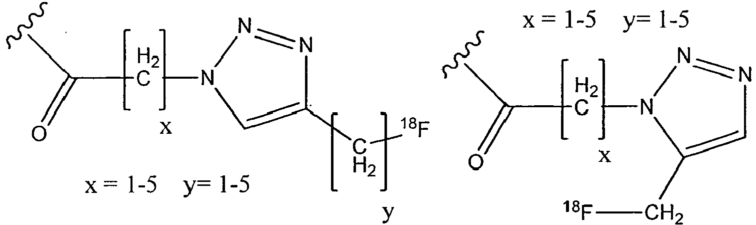 |
| | 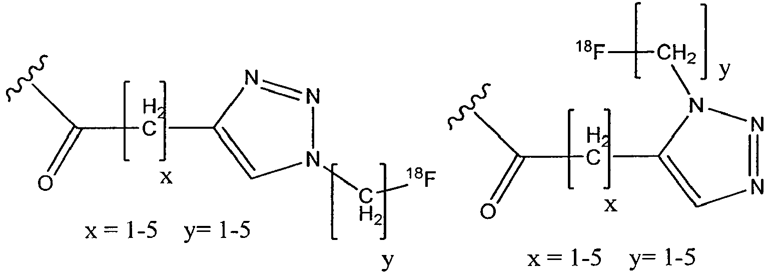 |
| | 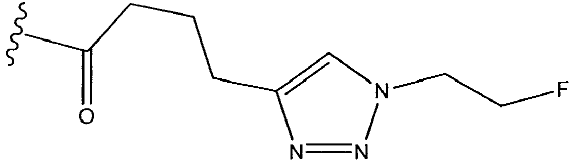 |
| | 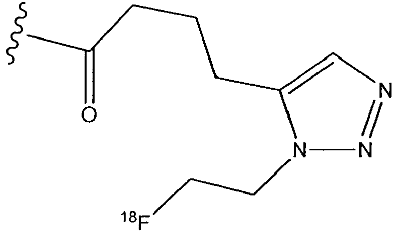 |
| | 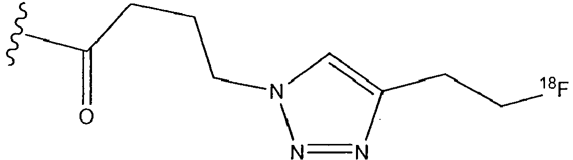 |
| | 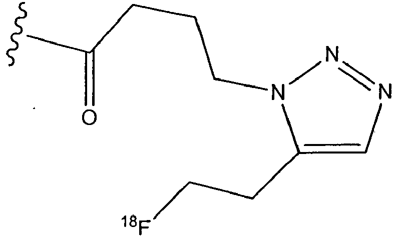 |
| | 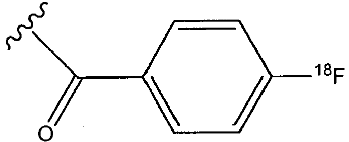 |
| | 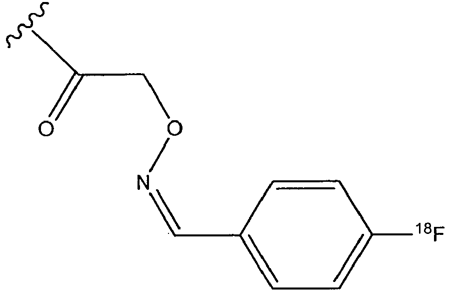 |
| | 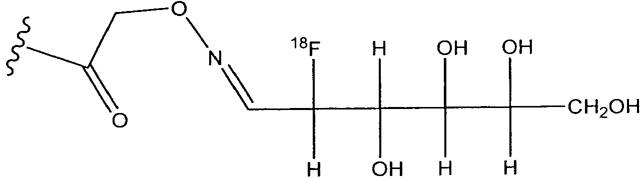 |
| | 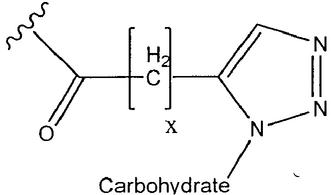 |
| | 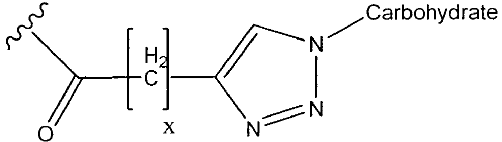 |
| | With: |
| | x = 1-5 |
| | Carboydrate : |
| | 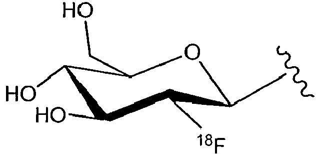 |
| | 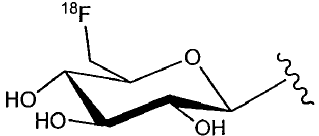 |
| | 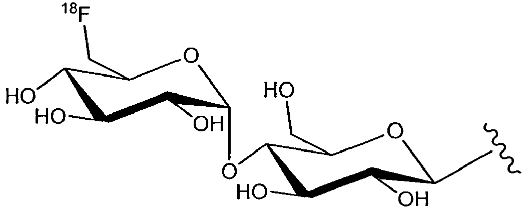 |
| | 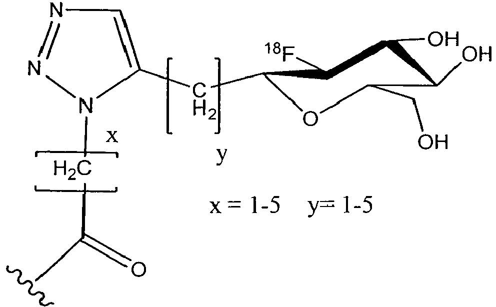 |
| | 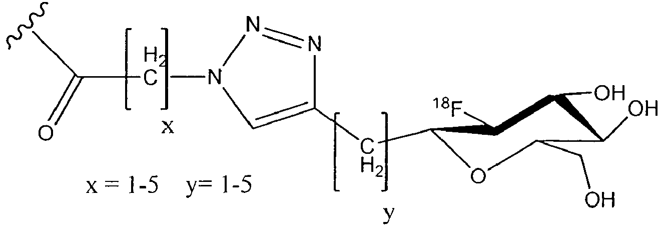 |
| | 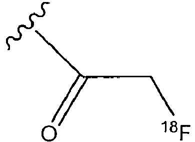 |
| | 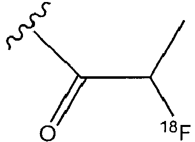 |
| | 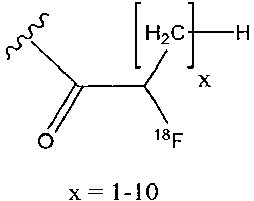 |
| | 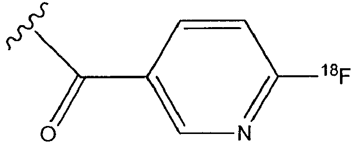 |
| | 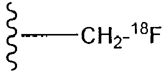 |
| | 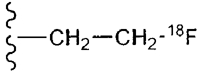 |
| | 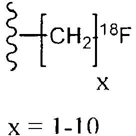 |
| | 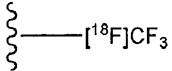 |
| | 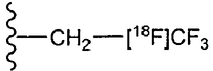 |
| | 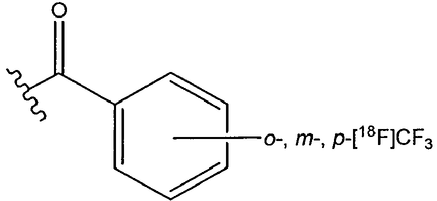 |
| | 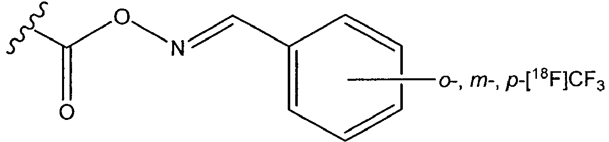 |
| | 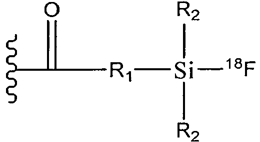 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 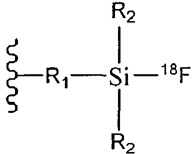 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 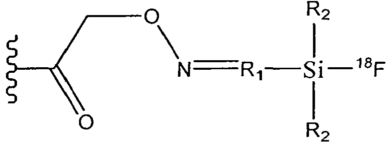 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 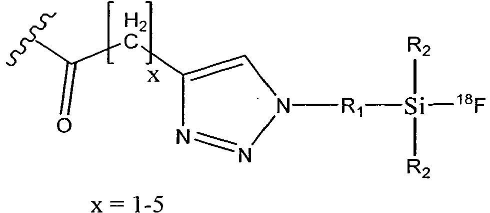 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 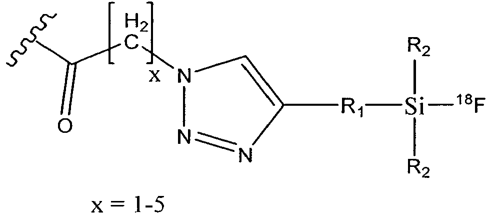 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 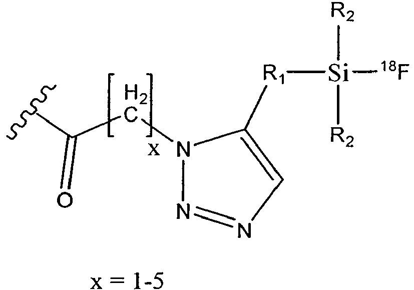 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |
| | 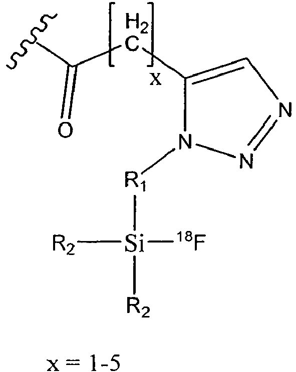 |
| | R₁: Any alkyl, aryl or arylalkyl linker |
| | especially methyl, 2-ethyl, 3-propyl, 2-,3-,4-phenyl, 2-,3-,4-phenylmethyl, 2-,3-,4-phenylpropyl |
| | R₂: Any alkyl or aryl group |
| | especially methyl isopropyl, *tert*-butyl, phenyl or 1-naphtyl |

2. The compound of claim 1 having the structure R'-Linker-R with R'= ¹⁸F-Tag and R = Glu-Urea-Lys: 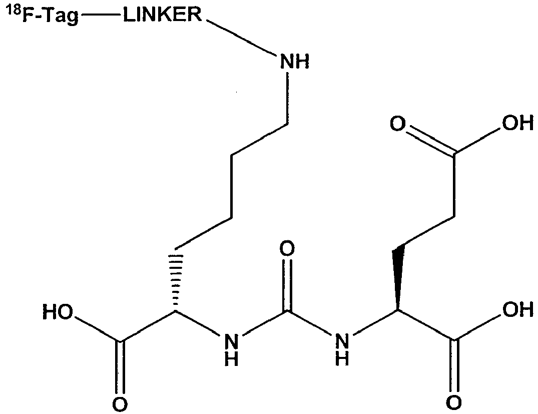 wherein the linker is selected from the group

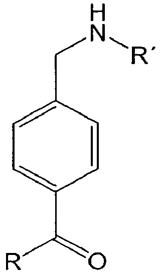

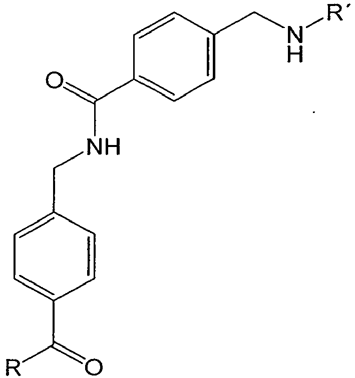

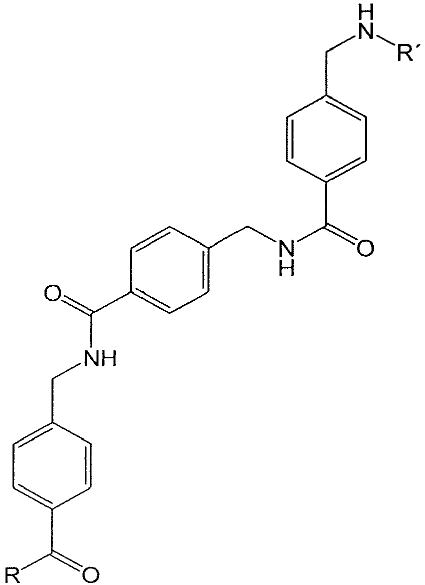

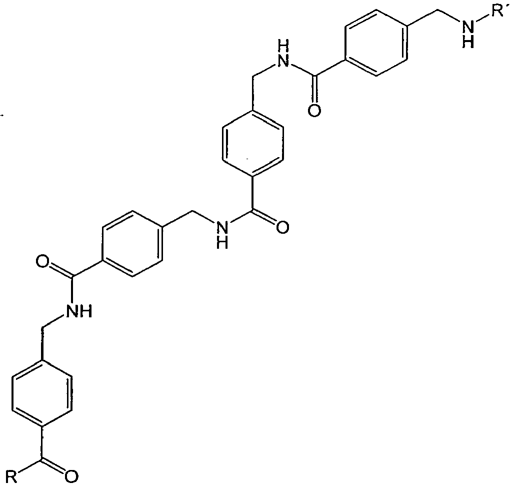

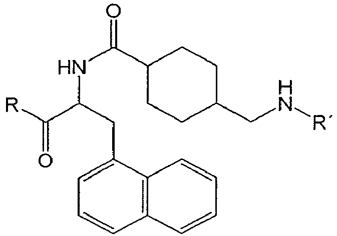

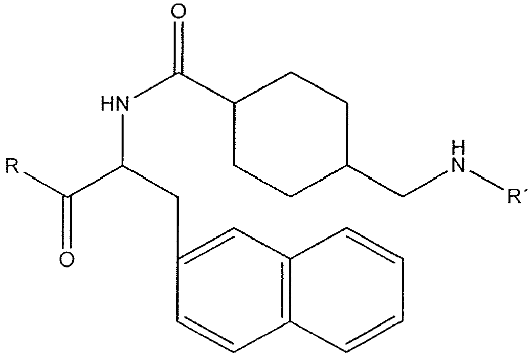

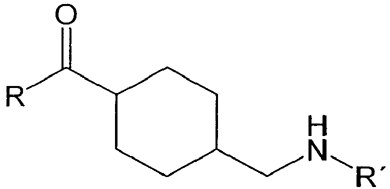

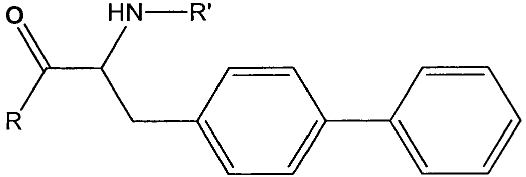

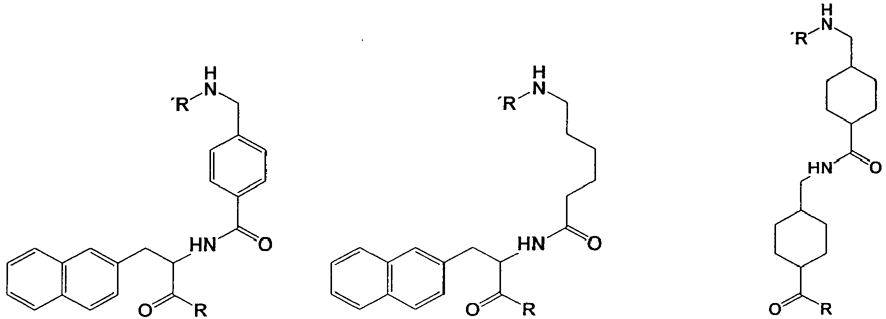

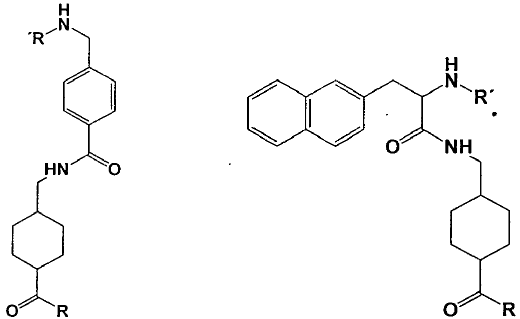

3. The compound of claim 1 or 2, selected from the following:

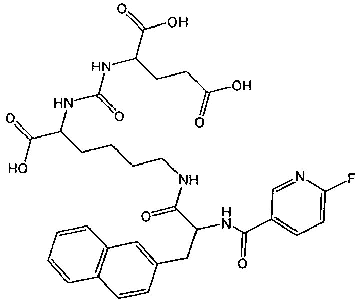

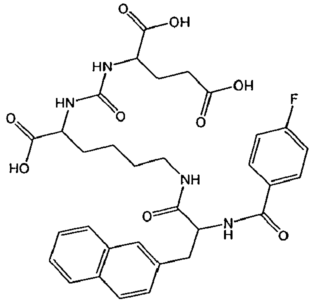

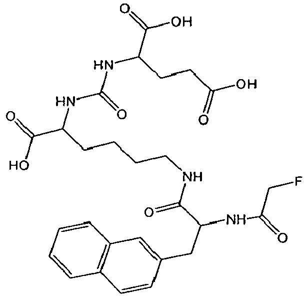

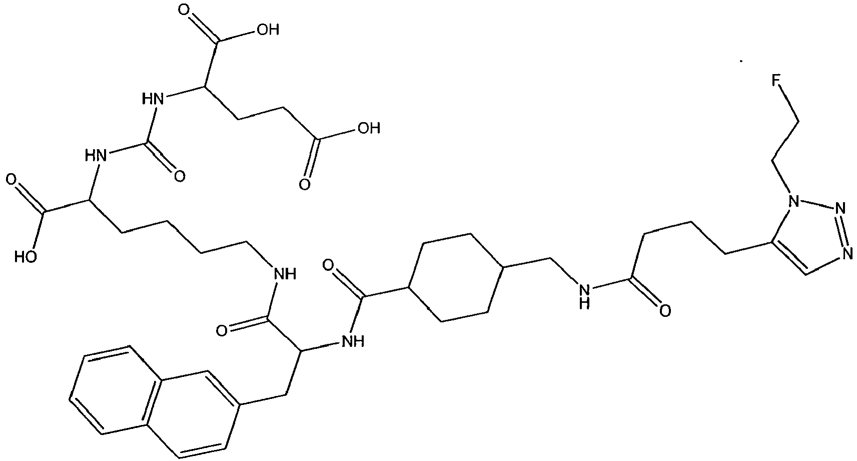

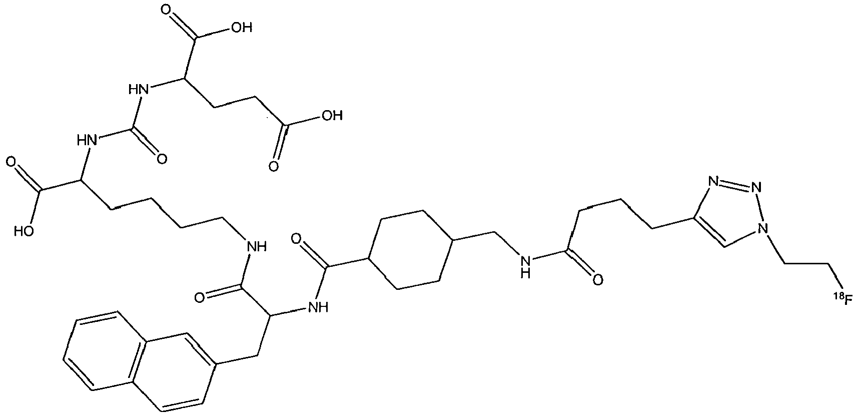

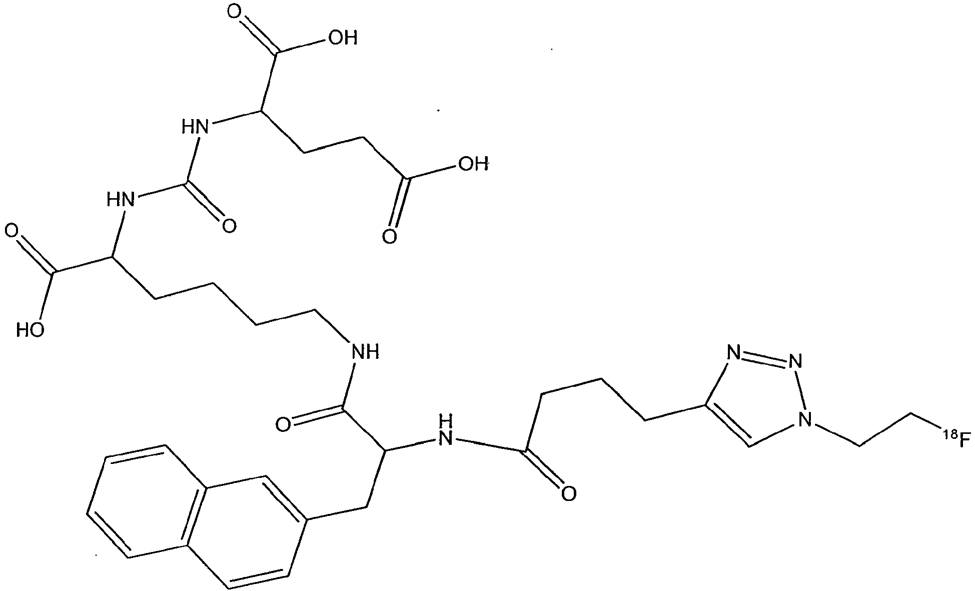

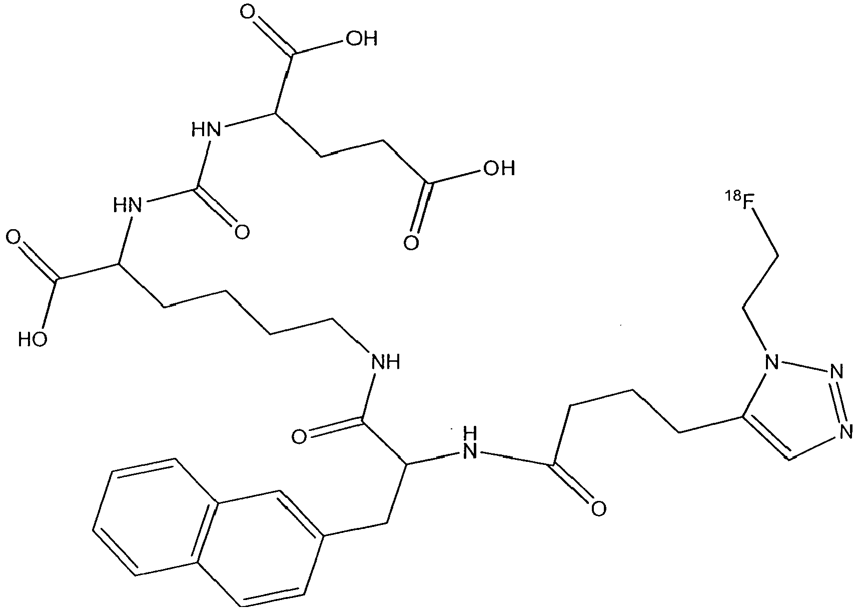

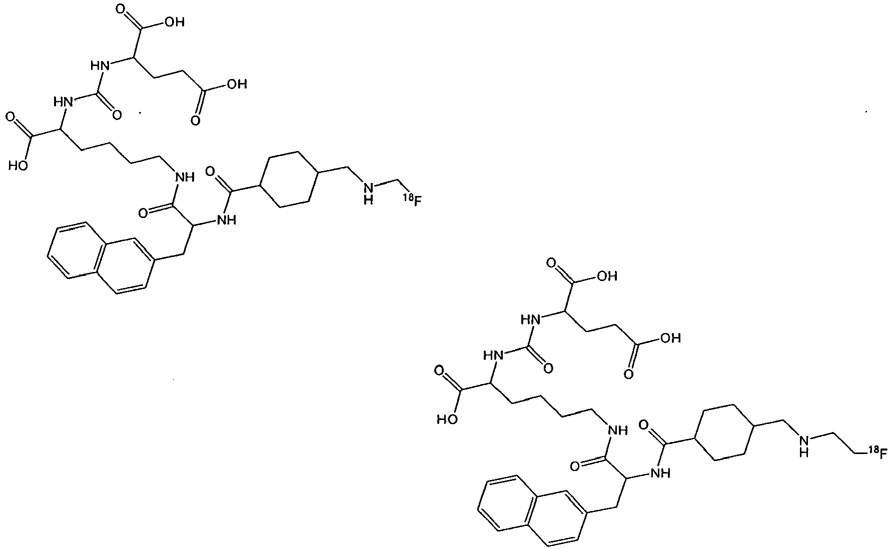

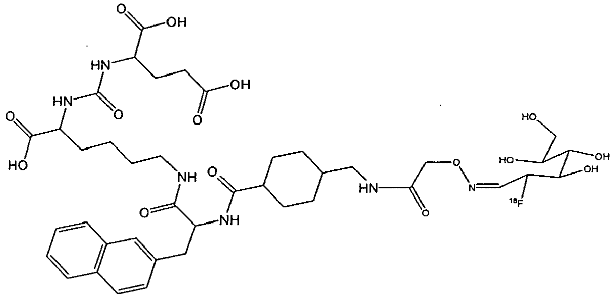

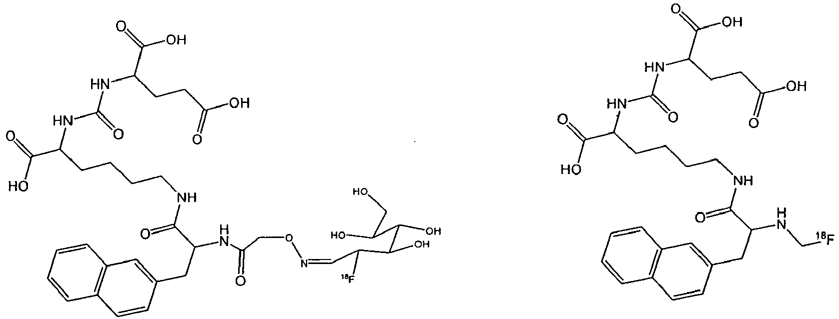

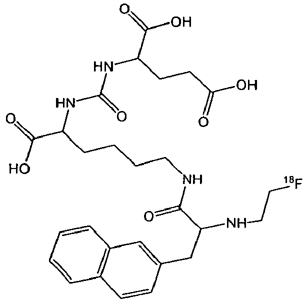

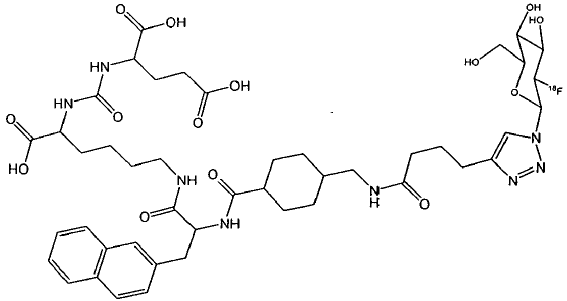

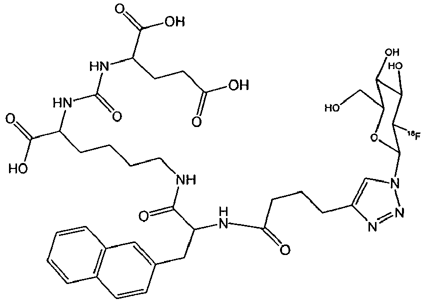

4. A pharmaceutical composition comprising a compound of any of claims 1 to 3, or a pharmaceutically acceptable salt, or ester thereof, and a pharmaceutically acceptable carrier.

5. Compound of any of claims 1 to 3 for use in a method of imaging in a patient.

6. Compound of any of claims 1 to 3 for use in a method of diagnosing prostate cancer and/or metastasis thereof.

7. Compound of any of claims 1 to 3 for use in a method of treating prostate cancer. and/or metastasis thereof.
